# EUROPEAN PATENT APPLICATION

(11) **EP 4 064 007 A1**
(43) Date of publication of application: **28.09.2022**
(21) Application number: 21164037.0
(22) Date of filing: 22.03.2021
(51) Int. Cl.: G06F 3/01, A61B 5/11

(54) **TACTILE INTERACTION MONITORING**

(71) Applicant: Koninklijke Philips N.V., 5656 AG Eindhoven (NL)
(72) Inventor: WRIGHT, Christopher John, 5656 AE Eindhoven (NL); LAWRENSON, Matthew John, 5656 AE Eindhoven (NL)
(74) Representative: Philips Intellectual Property & Standards

(57) **Abstract**

A thermal monitoring system (10) is for monitoring a touch interaction between two persons based on heat tracking. A thermal stimulus generator (12) is used to generate coded or patterned thermal stimuli (14). The thermal stimulus generator is arranged, to generate the thermal stimuli so that when the user touches a subject with one of a defined set of touching areas of the hand, one of the thermal stimuli interposes the touch interaction and applies the thermal stimulus to the skin of the user. This leaves an impression of the thermal pattern or code (16) embodied by the thermal stimulus on the skin of the subject. Different touching locations of the hand are uniquely associated with thermal stimuli with different thermal patterns or codes, allowing for retrospective tracking of touch events based on thermal monitoring of the touch interaction.

## Description

### FIELD OF THE INVENTION

The present invention relates to a system and method for monitoring a tactile interaction between at least two persons.

### BACKGROUND OF THE INVENTION

A growing area of interest is automated patient diagnosis and automated monitoring of patient-doctor interactions, for example during a medical consultation. It is known for example to use visual imaging technology to digitally image a skin lesion or other visible patient presentation, and to use artificial intelligence or other computer technology to derive a diagnosis. This can help to make diagnosis more objective, or to supplement or augment the diagnosis provided by the doctor.

It is also known to use cameras placed in doctor consultation rooms to monitor patient-doctor interactions visually. This can be used in providing supplementary diagnostic information, and also as part of training or professional development of a doctor, for instance by giving feedback on technique.

Known systems have limitations.

First, with a fixed camera system, a view of the patient-doctor interaction can often become obscured, for instance if the doctor moves in the way of the camera field of view. This leads to gaps in the data and limits the useful information that can be obtained. Additional cameras can be added, but this adds greatly to the computational complexity of processing the image data and deriving useful information.

Furthermore, there are inherent limitations in the information a visual-based system can provide. It can only provide direct diagnostic analysis of clinical presentations which are visual in nature, e.g. skin lesions, rashes, swelling, redness etc. Conditions requiring tactile examination, e.g. lumps or pain in structures beneath the skin, are difficult to monitor.

A camera based system can be used to track touch interactions visually. However, the processing required to accurately detect and track each incidence of touch between the doctor's hands and the patient is complex and computationally expensive. This is further exacerbated if a view becomes obscured and processing must be applied to correct for data gaps.

Systems have been proposed for directly detecting finger touch interactions between a doctor and a patient, with use of a glove containing pressure or contact sensors. However, it is difficult for isolated sensors on a glove to determine all properties of a physical interaction. Accurate touch dynamics are particularly difficult to detect. Detecting a position of each touch event in relation to the patient's body is also difficult.

An improved approach to monitoring patient-doctor tactile interactions would therefore be of benefit.

### SUMMARY OF THE INVENTION

The invention is defined by the claims.

According to examples in accordance with an aspect of the invention, there is provided a system for monitoring a tactile interaction between a user and a subject, the tactile interaction comprising touching of one or more locations on tissue surfaces of the subject's body with one or more touching areas of the user's hands, the system comprising:
a thermal stimulus generator adapted to generate active thermal stimuli which are arranged to be applied to touched locations of the subject's body upon touch of those locations by the user,
   wherein the thermal stimuli are spatially coded or patterned, whereby the application of a thermal stimulus thereby transfers an impression of a thermal pattern or code of the stimulus to the subject's tissue surface, and
   wherein differently patterned or coded thermal stimuli are uniquely associated with different touching locations of the user's hand;
a thermal monitoring apparatus for acquiring thermal imaging data of the tactile interaction, for use in detecting transferred thermal patterns on the tissue surfaces of the subject;
a processing unit configured to reconstruct properties or dynamics of the tactile interaction based on data from the thermal monitoring apparatus.

Embodiments of the invention are based on the concept of using the residual heat transferred from the fingers during touch to track and monitor touch interactions. This residual heal can persist for a window of time after the touch event, e.g. 10s at normal body temperature. This can be detected on the touched object using thermal imaging. This concept has been used to extract pin numbers from keypads for example.

Embodiments of the invention are based on extending this concept and generating artificial thermal stimuli which will interpose the user's hands and the subject being touched and leave a residual thermal signature. The thermal stimuli are uniquely patterned or coded depending upon which point on the user's hand is doing the touching making for simple and easy tracking of the touch interaction.

The application of the coded or patterned thermal stimuli to the subject leaves a thermal impression of the spatial pattern/code of the stimulus on the skin of the subject. Heat is transferred to or from the thermal generator upon application of the thermal stimulus to the subject. Thus each coded thermal stimulus has an associated thermal signature which can be detected by a thermally sensitive imaging device. In other words, the residual pattern of heat/cold is detectable by a thermally sensitive detector, e.g. a thermal camera.

In this disclosure, a touch event refers to a single touch between a particular area of the user's hand and a location on the subject. A touch interaction refers to a whole session of tactile interaction between user and subject, e.g. a doctor's consultation with a patient. Reference to a thermal pattern means the thermal pattern or code embodied by a particular patterned or coded thermal stimulus and transferred to the subject's skin upon touch.

The thermal stimuli created by the thermal stimulus generator may be generated at locations coincident with different locations of the user's hand(s). The thermal stimulus generator may be arranged so that during a touch event, a thermally active area of the generator forms a physical interface between the touching area of the user's hand and the touched location of the subject's skin. This thermally active area generates the thermal stimulus.

Thermally coded or patterned means that the thermal stimuli define a particular spatial distribution of heat across an active area. This may be a continuous heat distribution, but more preferably it is a spatial pattern with one or more gaps or discontinuities.

The thermal stimulus generator may be configurable in a non-deployed and a deployed configuration. In the deployed configuration, the thermal stimulus generator may be mounted or supported in a defined (e.g. fixed) spatial arrangement relative to touching areas of one or both hands of the user. The deployed configuration may have the generator mounted to at least a portion of the hand, wrist or arm in a predictable fixed or movable relation to the hand, such that thermally active areas of the generator, upon touch action by the user, physically interpose the touching areas and the touched location of the subject.

The thermal stimulus generator may be arranged to be carried by, or worn on, one or both hands of the user. The stimulus generator may be a hand worn device. The generator be a single unitary unit, e.g. a glove, or may be composed of multiple pieces, e.g. a set of thermally active pads which adhere to different locations on the user's hands.

The thermal stimulus generator may be in the form of a glove.

The thermal stimulus generator may comprise a plurality of thermally active areas for being disposed, in operation, over different respective touching areas of the user's hand. For example, the thermally active areas are for overlying the touching locations of the user's hand(s).

The thermally active areas may interpose or interface touch interactions between the touching areas of the hand and the touched locations of the subject.

The thermally active areas may comprise thermally active elements which generate the patterned or coded thermal stimuli, and preferably wherein the thermally active elements define a spatial shape or arrangement which forms the spatial pattern of the thermal stimulus. The thermally active elements may be heating elements, for example resistive heating elements, e.g. resistive wires. These may be bent, shaped or arranged in a particular spatial pattern for example.

Alternatively, the thermally active elements may be cooling elements.

In accordance with one or more embodiments, the thermal patterns of the stimuli generated by the stimulus generator may be dynamically adjustable.

For example, each thermally active area may comprise a regular array of thermal elements, which are individually addressable to control a desired pattern.

In alternative examples, the thermal patterns of the stimuli associated with each user touching location may be fixed.

The reconstructing of the properties of the touch interaction may comprise identifying a set of touch events occurring during the monitored touch interaction, wherein each touch event comprises touching of a location on the subject's body with a particular touching area of the user's hand(s).

The reconstructing of the properties of the touch interaction may comprise determining for each touch event at least:
a location on the body of the subject which was touched; and
a touching area of the user's hand which was used to touch the subject.

Element (b) can be determined from the detected thermal pattern. Element (a) can be determined for example based on anatomical models and image processing.

The reconstructing of the properties of the touch interaction may comprise use of one or more machine learning algorithms. For instance, one or more machine learning algorithms may be trained to perform one or both of (a) and (b) above.

The reconstructing may further comprise determining a time of each touch event.

This may be done in some examples based on an internal clock of the processor or the monitoring apparatus and identifying a time stamp of an image frame at which a given pattern first appears in a given location.

In other embodiments, it can be done based on detecting a variation in a heat level (e.g. temperature) of each transferred thermal pattern as a function of time, based on a known initial heat level upon application. Time since its application can thereby be calculated. Timing of touch events can thus be reconstructed even in case a view of the subject becomes temporarily obscured.

In further embodiments, thermal patterns may vary as a function of time, with a known time-evolution. Time of application can be uniquely discerned in this case from the patterning or coding of the thermal pattern.

In accordance with one or more embodiments, the thermal monitoring apparatus may be configured to detect variation in a heat level of each transferred thermal pattern as a function of time. The heat level may refer to temperature for example.

In accordance with one or more embodiments, processing unit may be adapted to identify a heat level (e.g. temperature) of detected thermal patterns on the subject's skin and estimate a pressure of each touch event based on the detected heat level. More heat may be transferred with greater pressure for example.

In accordance with one or more embodiments, the system may include a tissue analysis module adapted to determine one or more properties of the subject's tissue based on the thermal imaging data and/or based on the reconstructed properties of the touch interaction.

The tissue analysis module may be configured to determine a measure of tissue perfusion at one or more locations based on heat dissipation dynamics of transferred heat patterns.

Examples in accordance with a further aspect of the invention provide a method for monitoring a tactile interaction between a user and a subject, the tactile interaction comprising touching of one or more locations on tissue surfaces of the subject's body with one or more touching areas on the user's hands, the method comprising:
generating active thermal stimuli at different locations of a user's hand, and which are arranged so as to be applied to touched locations of the subject's body upon touch of those locations by the user,
   wherein the thermal stimuli are spatially coded or patterned, whereby the application of a thermal stimulus thereby transfers an impression of the thermal pattern or code to the subject's tissue surface, and
   wherein differently patterned or coded thermal stimuli are associated with different touching locations of the user's hand;
acquiring thermal imaging data of the tactile interaction;
detecting transferred thermal patterns on the tissue surfaces of the subject based on use of the thermal imaging data; and
reconstructing properties or dynamics of the tactile interaction based on the thermal imaging data.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying schematic drawings, in which:
Fig. 1 outlines architecture of an example tactile interaction monitoring system;
Fig. 2 outlines components of an example thermal stimulus generator;
Fig. 3 shows an example thermal stimulus generator comprising a glove unit;
Figs. 4-5 illustrate different example patterned/coded thermal stimuli;
Fig. 6 illustrates transfer of thermal patterns by the thermal stimulus generator upon touch of the subject by a user;
Fig. 7 shows operation of the system within an examination room;
Fig. 8 shows a further example thermal stimulus generator, comprising adhesive finger portions for adhering to finger pads of the user; and
Fig. 9 outlines steps of an example tactile interaction monitoring method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a thermal monitoring system for monitoring a touch interaction between two persons based on heat tracking. A thermal stimulus generator is used to generate coded or patterned thermal stimuli. The thermal stimulus generator is arranged, in a deployed configuration, to have a defined spatial relationship with respect to a hand of the user (who is doing the touching), and is arranged to generate the thermal stimuli so that when the user touches a subject with one of a defined set of touching areas of the hand, one of the thermal stimuli interposes the touch interaction and applies the thermal stimulus to the skin of the user. This leaves an impression of the thermal pattern or code embodied by the thermal stimulus on the skin of the subject. Different touching locations of the hand may be uniquely associated with thermal stimuli with different thermal patterns or codes, allowing for retrospective tracking of touch events based on thermal monitoring of the touch interaction.

Embodiments of the invention thus provide a means for monitoring the properties of a touch interaction between a user and a subject (who is being touched). Heat signatures transferred by touch events persist for a window after time after each touch event. This allows, according to certain embodiments, for retrospectively identifying touch interactions even in case a view of the interaction becomes temporarily obscured.

Particular advantages include enabling objective measurement of physical diagnostic interactions with patients, and without a doctor needing to change their examination routines or workflows. Further advantages include enabling a simpler process of reconstructing the touch interaction compared with purely visual systems due to the provision of the recognizable codes which can be transferred thermally and persist thermally but would not be visible with standard visible-light imaging technology. The processing required is far simpler than purely visual approaches, since it does not require complex feature recognition algorithms - the unique thermal patterning of the stimulus is easier to detect than generic anatomical features for example. The system can be achieved with only low cost equipment.

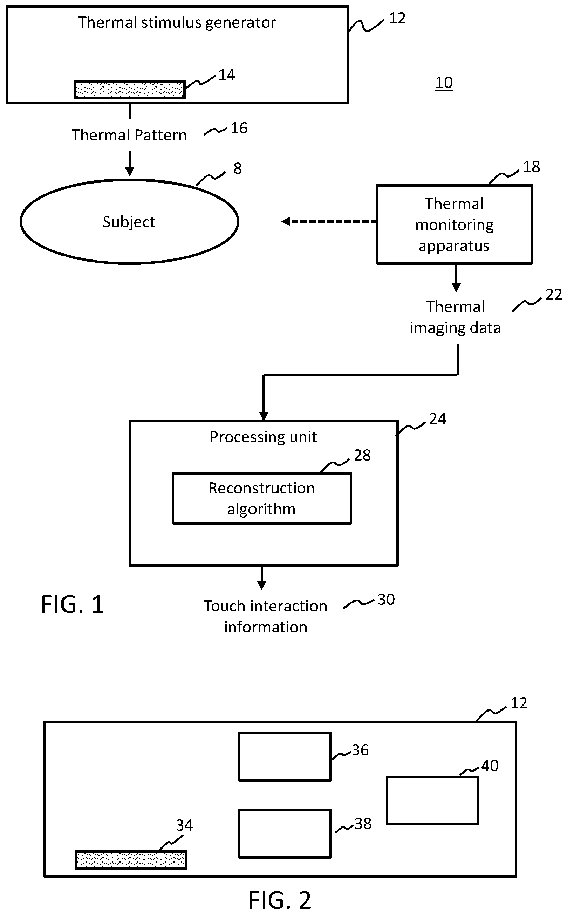

Fig. 1 schematically outlines the architecture of a tactile-interaction monitoring system 10 according to one or more embodiments.

The system 10 is for monitoring a tactile interaction between a user and a subject 8. The tactile interaction comprises touching of one or more locations on tissue surfaces of the subject's body with one or more touching areas of the user's hands.

The system 10comprises a thermal stimulus generator 12 which is adapted to generate active thermal stimuli 14 which are arranged to be applied to touched locations of the subject's body upon touch of those locations by the user.

The thermal stimuli 14 are spatially coded or patterned. The application of a thermal stimulus to the subject has the effect of transferring an impression of a thermal pattern 16 or code embodied by the stimulus to the subject's tissue surface. The tissue surface may be skin for example. The thermal stimulus generator 12 is arranged such that differently patterned or coded thermal stimuli are uniquely associated with different touching locations of the user's hand. In other words, the thermal stimulus generator is arranged so that different thermal codes or patterns are applied to the subject depending upon which of the touching areas is used to touch the user.

The system 10 further comprises a thermal monitoring apparatus 18 for acquiring thermal imaging data 22 of the tactile interaction. This data is for use in detecting transferred thermal patterns 16 on the tissue surfaces of the subject.

A processing unit 24 receives the thermal imaging data and is configured to reconstruct properties or dynamics of the tactile interaction based on the data. The processing unit may comprise one or more reconstruction algorithms 28 for use in processing the thermal imaging data to derive information 30 about the tactile interaction.

In accordance with one or more embodiments, the system may comprise a datastore. The datastore may store a record of the different coded thermal stimuli and their associated touching locations of the user's hand for use in reconstructing the touch interaction information 30. Alternatively, the datastore may store one or more algorithms, e.g. machine learning algorithms, the use in performing the reconstruction.

In operation, one or more thermal stimuli are generated on or in the thermal stimulus generator 12. The thermal stimulus generator is positioned or mounted in such a way that it physically interfaces between touching points of the user and the touched locations for the subject 8. For example, it is mounted or worn on the hand, with thermally active areas disposed over different locations on the user's hand.

The thermal stimulus generator 12 may be configurable in an idle configuration and a deployed configuration. In the deployed configuration, the thermal stimulus generator is mounted or supported in a defined (e.g. fixed) spatial arrangement relative to touching areas of one or both hands of the user. The deployed configuration may have the generator 12 mounted to at least a portion of the hand, wrist or arm in a predictable fixed or movable relation to the hand, such that thermally active areas of the generator, upon touch action by the user, physically interpose the touching areas and the touched location of the subject. The stimulus generator may comprise a support body which carries the thermally active areas, and which is comprised of one or multiple parts designed to be mounted in a defined spatial relationship to the user hand when deployed.

The user engages in tactile interaction with the subject 8 with the thermal generator 12 interfacing his or her touches with the patient. Thermal imaging data is collected by the thermal monitoring apparatus 18 during the touch interaction. The thermal imaging data is transmitted to the processing unit 24, through a wired or wireless connection. The processing unit processes the data, for example with one or more reconstruction algorithms 28. From this, touch interaction data 30 is generated representing information regarding the touch events during the touch interaction. This information may be transmitted to an external computer or processor for subsequent storage or analysis. For example it may be uploaded to a patient record management system and stored in the patient record for the user. It may be further analyzed to determine one or more clinical parameters as will be explained further below.

Fig. 2 schematically outlines components of an example thermal stimulus generator 12, some or all of which may be included in different embodiments. Thermal stimulus generator comprises a plurality of thermally active areas 34 which are adapted to generate thermal stimuli. Only one is visible in Fig. 2, however a plurality are included. The thermally active areas may include a set of one or more thermally active elements (not shown in Fig. 2), for example heating elements, which provide a source of heat for creating the thermal stimuli. The use of thermal elements is not essential. Other examples include use of thermally responsive materials which have intrinsic heat generation capabilities, responsive to electrical stimulation. The thermal stimuli refers to the actual pattern or spatial distribution of heat created actively by heat generating means comprised by the thermally active areas 34.

The stimulus generator 12 further includes a power source 36 for powering the heat generation by the thermally active area 34. This may be a local power store, such as a battery, which may be rechargeable. It may be a power inlet connector for connection to an external power source supply.

The stimulus generator 12 may further include a trigger mechanism 38 for triggering activation of thermal stimulus generation by the thermally active area 34. It may trigger or initiate delivery of energy from the power store to the thermally active area for example. This may be a manual user-activated button or switch which may mechanically complete the circuit or digitally complete a circuit. It may be a communication interface which receives a trigger signal from an external device, such as a user interface or a controller, e.g. the processing unit 24. It may be an automated trigger mechanism adapted to automatically activate generation of a thermal stimulus at a thermally active area 34 when the thermally active area engages with another physical surface. This may be a pressure sensitive element located in the thermally active area which completes a circuit responsive to pressure. The trigger mechanism may be configured to automatically trigger thermal generation responsive to the generator being put in a deployed configuration (e.g. mounted or attached to the user's hand). This may also be by means of pressure sensitive activation. Another option is use of a strain-sensitive element or switch incorporated in the body of the thermal generator, for example in cases in which a body of the thermal generator is formed of an elastic material, e.g. is in the form of a glove. The trigger mechanism may activate upon stretching of the glove when the glove is put on by the user. The trigger mechanism is not essential however.

Optionally, the thermal stimulus generator 12 may further include a controller 40 or processor which may be adapted to control activation of the thermally active elements or configure the thermal stimuli generated by the thermally active elements. In some cases, the thermal pattern or coding of stimuli may be dynamically configurable. The controller may function to control the thermal stimuli patterns or codes in some examples. Optionally a communication module may be included in the thermal stimulus generator 12 for communicating with an external device or controller, for example the processing unit 24.

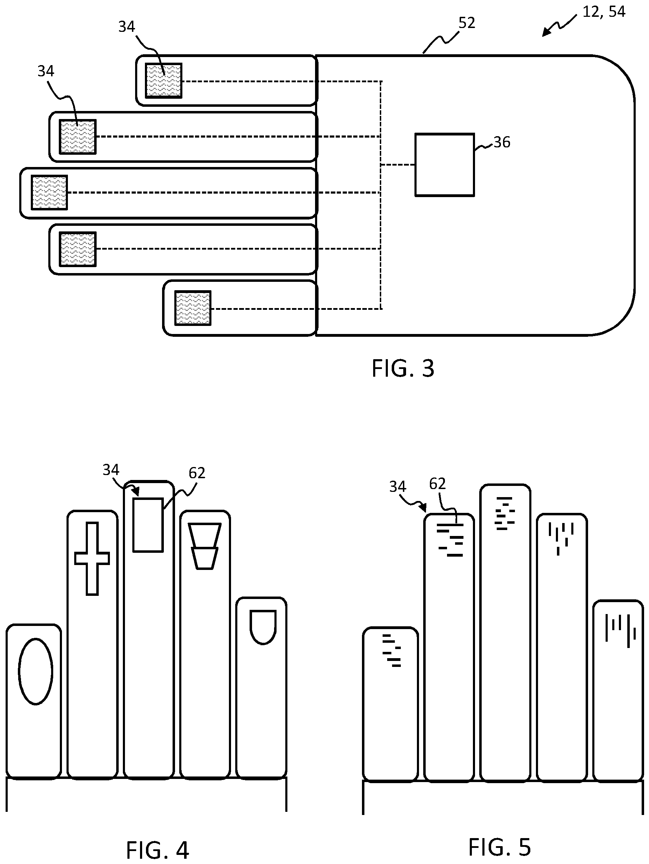

Fig. 3 shows an example thermal stimulus generator in accordance with one or more embodiments. In the illustrated example, the stimulus generator is provided in the form of a glove 54 worn by the user during touch interaction. The glove comprises a body 52 formed for example of an elastic fabric. The thermally active areas 34 are shown carried by or incorporated in the body of the glove. In the illustrated example, the thermally active areas are at the ends of the finger portions of the glove. They are positioned to coincide with the finger pads of the user when wearing the glove. In this case, the finger pads are the touching areas of the user. The thermally active areas may additionally or alternatively be located at other areas of the glove, for example additional or alternative locations on the fingers and/or within the palm portion of the glove. The thermal stimulus generator further includes a power source 36 for powering heat generation within the thermally active areas for creation of the patterned thermal stimuli. There may also be included a trigger mechanism (not shown).

In one or more examples, the body of the glove (or other form of thermal stimulus generator) may be composed of an elastic material such as Latex, Nitrile or any other suitable material. The material may in some examples be compressible such that the transfer of thermal energy from the thermally active areas to the patient intrinsically varies as a function of touch pressure. Thus, in this case, the body of the glove is a compressible membrane with variable thermal transmission properties as a function of applied pressure, which may permit later measurement of the applied pressure through detection of each level of the transferred thermal pattern on the subject.

Optionally, the material of the glove body 52 may be configured such that the thermal transfer varies in a non-linear way with applied pressure, which may make detection of applied pressure simpler and more accurate.

Although in the illustrated example of Fig. 3, the thermal stimulus generator 12 is in the form of a glove 54, other configurations are possible and will be discussed further later. The above features and comments can be applied to glove embodiments as well as other configurations of the thermal stimulus generator.

In general, the thermal stimulus generator 12 may include a body structure 52 which forms a carrier for the thermally active areas 34. The body structure may be comprised of one or multiple parts, connected to one another, or spatially separated from one another. As discussed above, the thermal stimulus generator may be configurable in an idle and deployed configuration, where the deployed configuration has thermally active areas in a defined fixed or movable relationship with respect to the hand of the user.

The thermal stimuli 14 generated by the thermally active areas 34 are configured to alter the subject's touched tissue (e.g. skin) temperature above or below the current level. Increased temperature may be achieved with heat generating means in the thermally active areas. The typical skin temperature of a human falls within a predicable range (e.g. approximately 33-38 °C), and thus heating-type thermally active areas can be designed to generate thermal stimuli at a temperature higher than the range of typical skin temperatures, to result in transfer of additional thermal energy. An alternative is to include cooling means to generate a cold thermal stimulus in a particular spatial pattern or coding.

For generating the thermal stimuli 14, the thermally active areas may comprise thermally active elements which generate the patterned or coded thermal stimuli. The thermally active elements may for example define a spatial shape or arrangement which forms the spatial pattern or coding of the thermal stimulus.

The thermally active elements may draw power from the power store to generate heat. They may for example comprise thermally conductive elements, for example resistive heating elements, e.g. resistive wires. These may be bent, shaped or arranged in a particular spatial pattern for example. This therefore allows for creating corresponding patterns of heat (the thermal patterns).

Fig. 4 shows an example of use of heating elements 62 within an embodiment in which the thermal stimulus generator 12 is in the form of a glove. In this case, the thermally active areas 34 are at the ends of the finger regions of the glove to coincide with the user's finger pads in use. The thermally active areas 34 comprise thermally active heating elements 62 which are bent or otherwise shaped to form specific recognizable shapes or patterns.

As shown, thermally active areas 34 located at different touching locations of the stimulus generator have thermal elements defining different respective spatial patterns or coding. In this way, the thermal signature from each touching location may be uniquely recognizable. For example, each finger of the glove may consist of a varying spatial arrangement of thermal elements 62, or thermal application pattern. In some examples, there may be multiple spatial stimulus patterns within a single thermally active area. This allows more fine-grained recognition of which regions of a particular user touching area has been used in a touch event. For example there may be multiple different patterns, or spatially varying patterns, embodied at the location of each finger pad, or otherwise adjacent areas of the thermal stimulus generator.

Fig. 5 shows a further example use of thermal elements 62 to generate pattered thermal stimuli. In this example, the patterns define a particular configuration of lines and dots which forms a coded thermal signature.

Preferably, respective linear sections or portions of the thermal elements are spaced from one another to prevent parts of a thermal pattern bleeding into one another too quickly when transferred to a user's skin. This ensures that each pattern on the skin remains resolvable within the time period that the thermal signal will be observable. For example, there may be a minimum spatial separation between the heated sections a thermal element.

The minimum separation may be defined based on an estimated value for the speed of thermal diffusion in patient skin. Thermal diffusion between strands of the thermal pattern reduce the ability to resolve thermal patterns over time. By way of example, the spacing may be configured such that the thermal patterns remain resolvable, after application the skin, for a time period of at least 5 seconds and preferably at least 10 seconds.

The thermal elements 62 may be controlled to heat to a level so as to increase the skin temperature of the subject without being uncomfortable. By way of non-limiting example, the elements may be heated to a temperature of between 35-50 °C.

In accordance with one or more embodiments, spatial patterning of the thermal stimuli may be configured so as to have a variable shape or appearance at a differential spatial locations across the areas of the pattern or code. This allows for distinguishing which part of the touching area has been touched against the user's skin. For example, a cross pattern may be used, where the portion of the pattern which is visible on the patient's skin would be used to determine which part of the thumb pad the user was pressing with.

In accordance with one or more embodiments, the thermal patterns 16 of the stimuli 14 generated by the stimulus generator 12 may be dynamically adjustable.

For example, each thermally active area may comprise a regular array of thermal elements, and wherein the thermal pattern may be controllable based on controlling the pattern of signals issued to the array of thermally active elements, e.g. selectively activating only a subset of the elements which define a desired pattern or code, or varying a heat level of different elements.

In some examples, the thermal patterns may be dynamically varied as a function of time over the course of a touch interaction session, so that timings of touch events can be identified.

Additionally or alternatively, they may be dynamically varied during a single touch event to enable additional properties of the touch event to be determined.

A controller 40 may be included in the thermal stimulus generator arranged to control the variable patterns as a function of time. Pre-defined pattern-time evolution functions for each thermally active area 34 may be stored in a local datastore of the stimulus generator 12 and used to define how the patterns are varied over time by the controller 40.

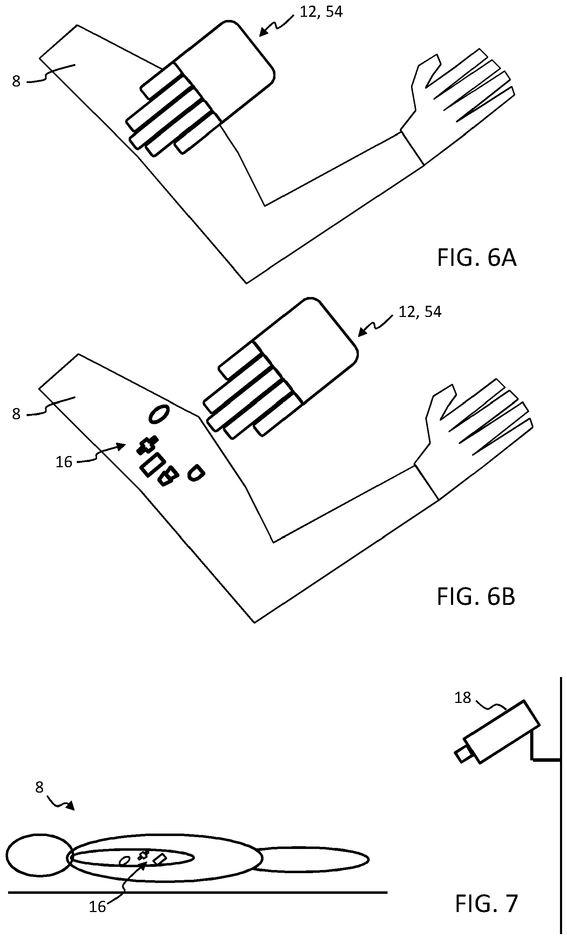

Use of the apparatus is illustrated schematically in Fig. 6. Fig. 6 a shows a user touching a subject 8 while the stimulus generator 12 (in this example in the form of a glove 54) is in the deployed position on their hand. Fig. 6B shows the thermal patterns 16 left on the skin of the subject after release of the user's hand.

Fig. 7 shows a setup of the system within a space, e.g. an examination room of a doctor. Thermal monitoring apparatus in the form of a thermal imaging camera 18 is shown mounted to a wall of the space, and wherein a field of view of the thermal camera covers a region containing the subject 8 and in which the touch interaction with the subject will occur.

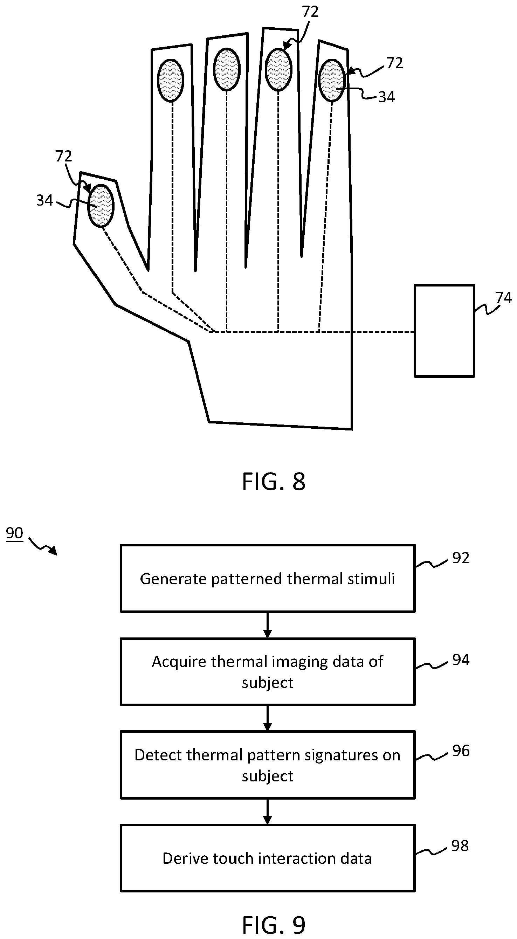

Although a thermal stimulus generator 12 has been discussed above in the form of a glove 52, other configurations are also possible. Fig. 8 shows a further example of a thermal stimulus generator in accordance with one or more embodiments. The stimulus generator in this case is formed of multiple generator portions 72, each of which forms a thermally active area 34. The multiple portions may for example each be formed of a membrane arranged to be adhesively coupled to areas of the user's hand, for example the finger pads of the user's hands as depicted in Fig. 8. The thermally active area of each of the multiple stimulus generator portions 72 is connected to a local power source or controller 74. The connection may be wired connection or wireless energy coupling may be considered, for example inductive energy coupling. The local controller or power store 74 may be mounted in a wearable unit, for example a wrist mountable unit, for the convenience of the user. Optionally, a communication interface may further be included for receipt of control instructions regarding activation of the thermally active areas 34 or the patterns or codes of the thermal stimuli to be generated by the thermally active areas.

As discussed above, the system includes a thermal monitoring apparatus 18. This is used to collect thermal imaging data of the touch interaction between user and the subject. This may for example comprise one or more thermal cameras located in a space in which the touch interaction is to take place. These are operable to detect radiant heat signals in the infrared frequency band. They may therefore be used to detect the residual thermal patterns transferred onto the skin of the subject 8 by the thermal stimuli 14 through the emission of infrared radiation by these thermal patterns.

Once the thermal imaging data 22 of the touch interaction has been acquired, a processing unit 24 reconstructs properties or dynamics of the touch interaction. The reconstruction procedure will now be described in more detail.

In general, the reconstructing of the properties of the touch interaction may comprise determining for each touch event at least:
a location on the body of the subject which was touched; and
a touching area of the user's hand which was used to touch the subject.

Optionally, the processing unit 24 may also be configured to derive an estimate of the pressure at which a thermal stimulus was applied to the subject.

Element (b) can be determined from the detected thermal pattern. In a simple example, this can be done using a pre-stored reference dataset which records the different thermal patterns and corresponding touching locations on the user's hand (or locations on the thermal generator). Well known image processing algorithms, e.g. shape matching algorithms, can be used to identify the shapes or patterns of the different thermal stimuli within the acquired image data. These can then be compared with the reference dataset to identify which of the thermal stimuli they correspond to, and therefore which touching area of the user's hand was used to generate each pattern.

In another example, a machine learning algorithm may be used to detect the thermal patterns in the thermal imaging data 22 and to decode the thermal signatures and identify the user touching location.

Element (a) may be determined using image processing algorithms and for example using a reference anatomical model to identify an anatomical location on a subject's body at which a particular thermal pattern has been detected. Image processing techniques for performing such anatomical analysis are known, and may employ for example segmentation techniques or shape matching techniques.

Element (a) may comprise determining an anatomical location on the subject's body, or, more generally, a location within a co-ordinate system or map defined relative to the body.

Alternatively, element (a) may be derived using a machine learning algorithm for example trained to identify anatomical locations within thermal imaging data.

By way of example, according to one or more embodiments, the reconstruction algorithms 28 of the processing unit 24 may include a machine learning algorithm which has been trained to recognize the thermal patterns of the thermal stimuli 14 and to add identifier flags or labels to the thermal imaging data 22 output from the thermal monitoring apparatus indicative of the recognized patterns. The output of this machine learning algorithm will be referred to as flagged or labelled thermal data. It may thus receive the thermal imaging data as an input and provide the flagged or labelled thermal data as its output.

The reconstruction algorithms 28 may additionally or alternatively include a machine learning algorithm which has been trained to recognize the anatomical location of the identified thermal patterns within the thermal imaging data. This may optionally be the same machine learning algorithm as above. For example, it may take as its input the flagged or labelled thermal data mentioned above. Alternatively, flagged or labelled imaging data may be provided from a different algorithm, e.g. an algorithm which uses a simple-lookup table to identify the patterns, and this data is provided as the input to the machine learning algorithm.

Optionally, a machine learning algorithm may be included which has been trained to estimate the physical pressures applied during each touch interaction event, based on the flagged thermal data, either provided by a first machine learning algorithm (where one is included), or from a different source, as discussed in the preceding paragraph. The pressure may for example be discerned from heat level of the thermal signatures in the thermal imaging data for example.

The reconstructing may further comprise determining a time of each touch event. This can be done in different ways.

This may be done in some examples based on an internal clock of the processor or the monitoring apparatus and identifying a time stamp of an image frame at which a given pattern first appears in a given location. A time at which a thermal pattern is first detected on the subject's skin may be identified as the time of the corresponding touch event.

In other embodiments it can be done based on detecting a variation in a heat level (e.g. temperature) of each transferred thermal pattern as a function of time. For example, an initial temperature of the transferred pattern may be known, e.g. from control settings or parameters of the thermal stimulus generator. The processor detects the temperature of the transferred thermal pattern on the subject. From this, the processor may calculate, based on the difference in temperature, and known thermal transfer or dissipation properties of the subject's skin, an estimated time at which the thermal pattern was initially applied to the skin. This allows the processor to reconstruct timing of touch events even in case a view of the subject becomes obscured for a temporary period during the tactile interaction.

In further embodiments, a time of the touch interaction may be determined in a different way. For example, in some embodiments, the thermal pattern associated with each touching location of the user's body may be dynamically varied as a function of time. Here, each thermally active area is actively controllable to generate a time-variable thermal patterning. This can be varied with a known evolution as a function of time, such that each thermal pattern is uniquely associated with both a particular touch location on the user's hand(s) and a particular window of time for which the pattern was active.

In further embodiments, a time of the touch interaction may be determined based on use of an additional sensing or monitoring system. For example a separate imaging system may be used which monitors the interaction through visual analysis, and enables timings of touch events to be detected and registered or calibrated with the touch events detected using the thermal monitoring apparatus.

In accordance with one or more embodiments, the processing unit 24 may be adapted to identify a heat level (e.g. temperature) of detected thermal patterns on the subject's skin and estimate a pressure of each touch event based on the detected heat level.

For example, the processing unit may estimate the pressure based on the measured heat level of the transferred thermal pattern, and based on knowledge of a heat level of the thermal stimulus corresponding to said pattern. (i.e. an initial application heat). It may also be preferable to identify a time of touch application, in case the moment of first detecting the pattern is not the same as the moment at which the pattern was applied (and so some heat may already have dissipated). This can be done using one or more of the approaches outlined above for detecting touch time.

Alternatively, in some examples, a pressure of a touch event may be determined based on an active pressure-dependent actuation mechanism in the thermal stimulus generator. For example, a pressure sensitive element or mechanism may be included in the thermal generator at each thermally active area which can sense the pressure of a touch event. The thermal (e.g. heat) level of the thermal stimulus may be controlled in dependence upon the pressure, with a known pressure-temperature relationship, such that a pressure of the touch can be determined from the detected heat level of the thermal pattern impression on the subject's skin.

Alternatively, the pressure-dependent heat level can be implemented in an analogue rather than digital fashion by using one or more components in the thermally active areas which are adapted to intrinsically vary a heating level dependent upon an applied mechanical pressure. This could include a pressure-sensitive resistor or other current limiting element, or could include intrinsic material properties of the thermally active area, for example materials with pressure-dependent electrical or thermal conductance properties.

As mentioned above, in some examples, one or more machine learning algorithms may be used for reconstructing the properties or dynamics of the touch interaction.

A machine-learning algorithm is any self-training algorithm that processes input data in order to produce or predict output data. Here, the input data may comprise thermal imaging data 22 from the thermal monitoring apparatus 18 and the output data may comprise reconstructed properties or parameters of the touch interaction, e.g. user touching location, touched location of the subject's body, pressure of touch, and duration of touch.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

A process for training machine learning algorithms for use in embodiments of this invention will now be discussed.

A training system may be used. The training system may comprise a database (training database) of training data. The training data may comprise sample thermal imaging data of the same format as will be acquired by the thermal monitoring apparatus 18 during monitoring of touch interactions. The sample thermal imaging data is labelled with ground truth touch interaction information that matches the touch interaction information 30 which it is desired the processing unit 24 output during operation. For example, this may include elements (a) and (b) discussed above (touching area of user and touched location of subject). Here, the labels may include bounding pixel coordinates within the image (of each thermal pattern), anatomical location of the bounded regions, and the identified thermal pattern. Other labels added to the data may include, but are not limited to: touch duration and touch pressure.

The ground truth labels may be acquired for example experimentally when compiling the training data.

A critic algorithm is included as part of the training system which scores the similarity of the labels generated by the machine learning algorithm during training and the ground truth labels.

A parametric adjustment algorithm uses the critic scores across a set of training data fed to the machine learning algorithm to determine an adjustment to make to the model parameters of the machine learning algorithm.

A supervised learning procedure is then implemented using the critic algorithm and the parametric adjustment algorithm, based on feeding the machine learning algorithm at least a portion of the sample training data and adjusting algorithm parameters to minimize deviation between the ground truth labels in the training data and the outputs of the machine learning algorithm.

By way of example, one example training process may run as follows.

The labelled training data may be split into a training set and a test set (e.g. 80% / 20% split is often used).

The input data entry of each training data element (the thermal monitoring data) is fed to the machine learning algorithm in turn. The critic algorithm may score the similarity of labels generated by the machine learning algorithm to the ground truth:
In the case of continuous variables such as touch time and pressure, a proportional error may be calculated.

A binary score value may be output for each metric by comparing the error to a pre-defined acceptable error threshold.

In the case of bounding coordinates (see above), the scoring may be done based on: calculating the average pixel location for the bounded area for the generated label, and scoring the output as 1 if it lies within the ground truth bounded area, and 0 if it lies outside.

In the case of the thermal pattern detection, a binary success value may be given for correct or incorrect match to the thermal pattern label.

The collection of score values may be used as a success metric for each of the one or more machine learning algorithms being trained.

For each algorithm, the parametric adjustment algorithm may alter the parameters (weights) of the machine leaning algorithm according to a known statistical optimization strategy such as a Monte-Carlo method.

The training process may be ended after a set number of iterations (e.g. 1000), or when the success scores do not improve over several iterations.

In accordance with one or more embodiments, a further analysis module may be included adapted to determine one or more clinical parameters or clinically relevant information based on the derived touch interaction information 30.

By way of example a tissue analysis module may be provided adapted to determine one or more properties of the subject's tissue based on the thermal imaging data and/or based on the reconstructed touch interaction information 30. For example, the tissue analysis module is configured to determine a measure of cutaneous tissue perfusion at one or more locations on the subject. This may for example be based on heat dissipation properties of transferred heat patterns at said one or more locations resultant from a touch event.

For example, the tissue analysis module may employ a rules-based algorithm which measures the change in a given thermal pattern detected in thermal imaging data 22 over time. An estimation of pattern sharpness may be made using standard image analysis techniques. A reduction in sharpness may be measured over time. Reduction in sharpness can be expected to occur to due heat dissipation of the pattern, which depends upon tissue perfusion characteristics. The rate of reduction may be then used to query a lookup table of perfusion properties. Optionally, additional factors such as room air temperature may be included in this measurement and lookup step to improve accuracy.

Additionally or alternatively to perfusion, the tissue analysis module may be adapted to determine a thermal decay rate of the transferred thermal patterns 16. Thermal decay rate means a rate of loss of heat of the thermal pattern (which includes transferal of heat to the environment and loss through transfer and dissipation into the skin). This can be determined for a given thermal pattern based on monitoring the thermal pattern for a window of time after it is first detected by the thermal monitoring apparatus 18. The change in thermal temperature over time may be matched to known decay function for example based on use of a lookup table or algorithm.

To determine an initial thermal transference at the time a thermal pattern is first applied would require observing the pattern when first applied. However, this is not always possible, for example is the view is obscured. A machine learning algorithm may be applied, trained to estimate an initial thermal transference and a time of touch based on a pattern of thermal decay over time. Alternatively, a rules based algorithm may be used. However, in this case there may need to be provided a separate monitoring system for independently detecting a time of the touch event. This could be done using a separate imaging apparatus. This might detect a full view of the touch event or may detect hand or arm patterns of the doctor which may act as visual cues. An alternative is to detect auditory cues, such as detecting a doctor speaking a particular phrase, e.g. "Can you feel here". The direct sound of the touch event might even be detected in some examples.

To determine full dynamics of a given touch event, the thermal decay function (perfusion and conduction to air/other tissue), time of initial touch, and initial thermal transference may each be determined. Methods for detecting each of these have been discussed above. In some examples, a machine learning algorithm can be used which is trained to determine estimated values for all of these properties, based on the thermal imaging data. Through appropriate training, a machine learning algorithm is able to deconvolute these different parameters from the thermal imaging data based on subtle clues in the imaging data.

In accordance with one or more embodiments, the processing unit 24 may be further adapted to apply a noise removal procedure or correction procedure to account to changes in skin temperature due to environmental factors after a thermal pattern has been applied. For example, the subject's skin temperature or perfusion may change over time due to having walked into a warm examination room from a cooler room. These changes lead to variations in thermal dissipation of the thermal pattern, and which may lead to inaccuracies in the determination of thermal perfusion properties. To compensate, the processing unit may use the thermal imaging data to detect any global or regional changes to perfusion over time (i.e. changes occurring over the whole of a given area), and use this as a corrective factor when estimating the initial thermal conditions of a touch event.

Examples in accordance with a further aspect of the invention provide a method for monitoring a tactile interaction between a user and a subject, the tactile interaction comprising touching of one or more locations on tissue surfaces of the subject's body with one or more touching areas on the user's hands.

Steps of an example method are outlined in block diagram in Fig. 9.

The method 90 comprises generating 92 active thermal stimuli at different locations of a user's hand, and which are arranged so as to be applied to touched locations of the subject's body upon touch of those locations by the user. The thermal stimuli are spatially coded or patterned, whereby the application of a thermal stimulus thereby transfers an impression of the thermal pattern or code to the subject's tissue surface. Differently patterned or coded thermal stimuli are associated with different touching locations of the user's hand.

The method 90 further comprises acquiring 94 thermal imaging data representative of the tactile interaction.

The method 90 further comprises detecting 96 transferred thermal patterns on the tissue surfaces of the subject based on use of the thermal imaging data.

The method further comprises reconstructing 98 properties or dynamics of the tactile interaction based on the thermal imaging data (and the detected thermal patterns).

Examples in accordance with a further aspect of the invention provide a computer program product comprising computer program code, the computer program code being executable on a processor or computer.

The code is configured to cause the processor to perform a method for monitoring a tactile interaction between a user and a subject, the tactile interaction comprising touching of one or more locations on tissue surfaces of the subject's body with one or more touching areas of the user's hands.

Active thermal stimuli are generated during the interaction at different locations of a user's hand, and which are arranged such that they are applied to touched locations of the subject's body upon touch of those locations by the subject,

The thermal stimuli are spatially coded or patterned, whereby the application of a thermal stimulus thereby transfers an impression of the thermal pattern or code to the subject's tissue surface.

Differently patterned or coded thermal stimuli are associated with different touching locations of the user's hand.

The method caused to be performed by execution of the code comprises:
receiving acquired thermal imaging data of the tactile interaction;
detecting, based on the thermal imaging data, transferred thermal patterns on the tissue surfaces of the subject; and
reconstructing properties or dynamics of the tactile interaction based on the thermal imaging data.

There are numerous possible applications for embodiments of the present invention.

In the case of clinical applications, acquired touch interaction information for a given consultation may be uploaded to case notes for the patient (subject). Manual data entry by the doctor regarding touch interactions can therefore be avoided.

In some examples, the touch interaction information can be used to provide diagnostic support for the clinician in relation to mechanical tissue properties (e.g. detection of a cancer, or of a skin condition). The touch dynamics and optionally any detected thermal dissipation information may be used for example by an AI algorithm to determine relevant patient tissue properties and suggest a diagnosis to the doctor.

In some examples support can be provided to the doctor for preventing the causing of pain to the subject, or providing objective data for diagnostics by detection of an exact touch interaction which elicited patient pain. The touch interaction information in this regard could be used for detection of pain thresholds of the subject, locations of sensitive areas and exact force dynamics which elicit pain (e.g. stretching, compression), and, more generally, for mapping pain sensitivities. Such functionality may be particularly useful in the case of diagnosis and characterization of neuropathy or sensitivity.

By uploading touch interaction information for each examination session with a patient, over time, mechanical changes in the patient condition can be monitored (e.g. progression speed of a skin condition). An analysis module or system may quantify the change in tissue properties over multiple patient visits. An AI algorithm may be used to determine diagnostically relevant information and suggest diagnoses to a doctor based on this information.

A further potential application of derived touch interaction data may be for providing training support for the clinician. For example there may be maintained a central database of clinician diagnoses and the exact touch interaction dynamics which led to those diagnoses. This database can be used to establish best practice in the community (e.g. highest diagnostic accuracy touch strategies) this can be used to generate training material guidelines for clinicians. In some examples, real-time touch interaction between a doctor and a patient may be compared against the best practice database and this may be used to generate targeted training materials for the clinician.

As discussed above, the system comprises a processing unit which makes use of one or more processors. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system for monitoring a tactile interaction between a user and a subject (8), the tactile interaction comprising touching of one or more locations on tissue surfaces of the subject's body with one or more touching areas of the user's hands, the system comprising:
a thermal stimulus generator (12) adapted to generate active thermal stimuli (14) which are arranged to be applied to touched locations of the subject's body upon touch of those locations by the user,
wherein the thermal stimuli are spatially coded or patterned, whereby the application of a thermal stimulus thereby transfers an impression of a thermal pattern (16) or code of the stimulus to the subject's tissue surface, and
wherein differently patterned or coded thermal stimuli are uniquely associated with different touching locations of the user's hand;
a thermal monitoring apparatus (18) for acquiring thermal imaging data (22) of the tactile interaction, for use in detecting transferred thermal patterns (16) on the tissue surfaces of the subject;
a processing unit (24) configured to reconstruct properties or dynamics of the tactile interaction based on data from the thermal monitoring apparatus.

2. A system as claimed in claim 1, wherein the thermal stimulus generator (12) is arranged to be carried by, or worn on, one or both hands of the user.

3. A system as claimed in claim 2, wherein the thermal stimulus generator (12) is in the form of a glove (54).

4. A system as claimed in any of claims 1-3, wherein the thermal stimulus generator (12) comprises a plurality of thermally active areas (34) for being disposed, in operation, over different respective touching areas of the user's hand.

5. A system as claimed in claim 4, wherein the thermally active areas (34) comprise thermally active elements (62) which generate the patterned or coded thermal stimuli, and preferably wherein the thermally active elements define a spatial shape or arrangement which forms the spatial pattern of the thermal stimulus.

6. A system as claimed in any of claims 1-5, wherein the thermal patterns (16) of the stimuli (14) generated by the stimulus generator (12) are dynamically adjustable.

7. A system as claimed in any of claims 1-6, wherein the reconstructing of the properties of the touch interaction comprises identifying a set of touch events occurring during the monitored touch interaction, wherein each touch event comprises touching of a location on the subject's body with a particular touching area of the user's hand(s).

8. A system as claimed in claim 7, wherein the reconstructing of the properties of the touch interaction comprises determining for each touch event at least:
a location on the body of the subject which was touched; and
a touching area of the user's hand which was used to touch the subject.

9. A system as claimed in claim 8, wherein the reconstructing of the properties of the touch interaction comprises use of one or more machine learning algorithms.

10. A system as claimed in any of claims 7-9, wherein the reconstructing further comprises determining a time of each touch event.

11. A system as claimed in any of claims 1-10, wherein the thermal monitoring apparatus (18) is configured to detect variation in a heat level of each transferred thermal pattern as a function of time.

12. A system as claimed in any of claims 1-11, wherein the processing unit (24) is adapted to identify a heat level of detected thermal patterns (16) on the subject's skin and estimate a pressure of each touch event based on the detected heat level.

13. A system as claimed in any of claims 1-12, wherein the system includes a tissue analysis module adapted to determine one or more properties of the subject's tissue based on the thermal imaging data and/or based on the reconstructed properties of the touch interaction, and
preferably wherein the tissue analysis module is configured to determine a measure of tissue perfusion at one or more locations on the subject based on heat dissipation properties of transferred heat patterns at said one or more locations resultant from a touch event.

14. A method for monitoring a tactile interaction between a user and a subject, the tactile interaction comprising touching of one or more locations on tissue surfaces of the subject's body with one or more touching areas on the user's hands, the method comprising:
generating (92) active thermal stimuli at different locations of a user's hand, and which are arranged so as to be applied to touched locations of the subject's body upon touch of those locations by the user,
wherein the thermal stimuli are spatially coded or patterned, whereby the application of a thermal stimulus thereby transfers an impression of the thermal pattern or code to the subject's tissue surface, and
wherein differently patterned or coded thermal stimuli are associated with different touching locations of the user's hand;
acquiring (94) thermal imaging data of the tactile interaction;
detecting (96) transferred thermal patterns on the tissue surfaces of the subject based on use of the thermal imaging data; and
reconstructing (98) properties or dynamics of the tactile interaction based on the thermal imaging data.

15. A computer program product comprising computer program code, the computer program code being executable on a processor or computer
wherein, the code is configured to cause the processor to perform a method for monitoring a tactile interaction between a user and a subject, the tactile interaction comprising touching of one or more locations on tissue surfaces of the subject's body with one or more touching areas of the user's hands,
wherein active thermal stimuli are generated during the interaction at different locations of a user's hand, and which are arranged such that they are applied to touched locations of the subject's body upon touch of those locations by the subject,
wherein the thermal stimuli are spatially coded or patterned, whereby the application of a thermal stimulus thereby transfers an impression of the thermal pattern or code to the subject's tissue surface, and
wherein differently patterned or coded thermal stimuli are associated with different touching locations of the user's hand;
wherein the method comprises:
receiving acquired thermal imaging data of the tactile interaction;
detecting, based on the thermal imaging data, transferred thermal patterns on the tissue surfaces of the subject; and
reconstructing properties or dynamics of the tactile interaction based on the thermal imaging data.
